# EUROPÄISCHE PATENTSCHRIFT

(11) **EP 0 740 938 B1**
(45) Veröffentlichungstag und Bekanntmachung des Hinweises auf die Patenterteilung: **25.07.2001**
(21) Anmeldenummer: 96106804.6
(22) Anmeldetag: 30.04.1996
(51) Int. Cl.: A61K 31/401

(54) **Verwendung von Prolin als einziger therapeutischer Wirkstoff zur Herstellung eines Arzneimittels zur Behandlung von rheumatischen Erkrankungen und von chronischen und traumatisch bedingten Schmerzzuständen**
Use of proline as the only therapeutic agent for the manufacture of a medicament for the treatment of rheumatic diseases and for the treatment of chronic and traumatic pain
Utilisation de la proline en tant que seul agent thérapeutique dans la fabrication d'un médicament pour le traitement des maladies rhumatismales et de la douleur chronique et tramatique

(30) Priorität: 05.05.1995 CH 130195
(43) Veröffentlichungstag der Anmeldung: 06.11.1996
(73) Patentinhaber: IPR-Institute for Pharmaceutical Research Riehen AG, 4125 Riehen (CH)
(72) Erfinder: Meyer, Hans, 4125 Riehen (CH); Segesser, Bernhard, Dr., 4053 Basel (CH)
(74) Vertreter: Goth, Helmut, Dr.

(56) Entgegenhaltungen:
- EP-A- 0 324 227
- EP-A- 0 715 850
- DE-A- 2 438 703
- DE-A- 2 507 223
- DE-A- 2 547 243
- DE-A- 3 538 619
- FR-M- 4 727
- GB-A- 2 171 302
- US-A- 4 414 202
- US-A- 5 198 465
- LIFE SCI., Bd. 16, Nr. 9, 1.Mai 1975, USA, Seiten 1345-1361, XP000609320 CHVAPIL M.: "PHARMACOLOGY OF FIBROSIS:DEFINITIONS, LIMITS AND PERSPECTIVES"
- PEDIATRIC DERMATOLOGY, Bd. 13, Nr. 1, Januar 1996 - Februar 1996, Seiten 58-60, XP000609491 JEMEC G.B.E. ET AL.: "TOPICAL TREATMENT OF SCIN ULCERS IN PROLIDASE DEFICIENCY"
- INFLAMMATION, Bd. 3, Nr. 3, Juli 1979, (USA), Seiten 225-233, XP000609486 MEYERS, B., E. ET AL: "THE EFFECT OF SELECTED AMINO ACIDS ON GELATIN-INDUCED INFLAMMATION IN ADULT MALE MICE"
- ARCH. DERMATOL., Bd. 122, Nr. 6, Juni 1986, (USA), Seiten 626-627, XP000609225 JIRO ARATA ET AL.: "EFFECT OF TOPICAL APPLICATION OF GLYCINE AND PROLINE ON RECALCITRANT LEG ULCER OF PROLIDASE DEFICIENCY"
- DATABASE WPI Section Ch, Week 9544 Derwent Publications Ltd., London, GB; Class B03, AN 95-340244 XP002021255 & JP-A-07 233 147 (KONICA GELATIN KK) , 5.September 1995
- DATABASE WPI Section Ch, Week 9550 Derwent Publications Ltd., London, GB; Class B05, AN 95-390226 XP002021256 & JP-A-07 267 855 (OTSUKA SEIYAKU KOGYO KK) , 17.Oktober 1995
- DATABASE WPI Section Ch, Week 8148 Derwent Publications Ltd., London, GB; Class B05, AN 81-88158D XP002021257 & JP-A-56 133 213 (FUMIIRI M) , 19.Oktober 1981
- DATABASE WPI ACCESSION NO.: 66-30125F TECPAN S.A. XP002021254

## Beschreibung

Die Erfindung betrifft die Verwendung von Prolin zur Herstellung von Arzneimitteln, die Prolin als einzigen Wirkstoff enthalten zur Behandlung von rheumatischen Erkrankungen und chronischen und traumatisch bedingten Schmerzzuständen.

Die L-Form von Prolin (2-Pyrrolidincarbonsäure) ist eine natürlich vorkommende Aminosäure und ist beispielsweise in grösseren Mengen in Kollagen enthalten. Elastin enthält ebenfalls Prolin in jeder neunten Aminosäure-Position. Prolin gilt als nicht-essentielle Aminosäure.

Eine Fülle von N-substituierten Derivaten von Prolin oder Prolin-enthaltenden substituierten Peptiden sind bekannt als ACE-Hemmer und werden bei der Bekämpfung von Bluthochdruck eingesetzt (z.B. Captopril, Moveltipril, Zofenopril, Lisinopril, Enalapril und Enalaprilat). Oxaceprol, ein weiteres N-Acylderivat, besitzt antiphlogistische, antirheumatische und wundheilungsfördernde Wirkung. Ferner wurden in EP-A-O 535 923 und EP-A-O 535 924 Indolderivate offenbart, die einen N-acylierten Aminosäurerest, z.B. einen Prolinrest, enthalten können und die die Leukotrien-Biosynthese inhibieren und antiasthmatische, antiallergische, antiphlogistische und zytoprotektive Eigenschaften besitzen sollen. In handelsüblichen Infusionslösungen zur parenteralen Ernährung ist Prolin gelegentlich als Adjuvans (z.B. in Kombination mit anderen Aminosäuren, Kohlenhydraten und Elektrolyten) enthalten. So wird beispielsweise in DE-A-35 38 619 ein Mittel zur Behandlung von Krebs und Viruskrankheiten beschrieben, das neben mindestens einem 3-, 4- oder 5-Isomeren von Hydroxyprolin zur Erhöhung der Wirkung weitere Aminosäuren, wie Prolin, Valin, Alanin, Lysin, Hydroxylysin, Glycin, Cystein und Cystin enthalten kann. In DE-A-24 38 703 wird ein antirheumatisches Mittel beschrieben, das aus einer Mischung von 15 speziell genannten Aminosäuren besteht, wobei der Gehalt an jeder Aminosäure genau spezifiziert wird. Der Gehalt dieses Mittels an Prolin beträgt 14,9%. DE-A-25 47 2443 betrifft ein spezielles Verfahren zur Herstellung des in DE-A-24 38 703 beschriebenen Mittels. In US-A-5,198,465 wird ein Mittel zur Behandlung von Mangel an Vorläufern für die Kollagensynthese beschrieben, das frei von Hydroxyprolin und Hydroxylysin ist, und das zu 10 - 50% aus Prolin, 10 - 50% Glycin, 1 - 50% aus Vitamin C und zu 1 - 20% aus einer aus α-Ketoglutarsäure, Methionin, Cystein, Cystin und Valin ausgewählten Verbindung besteht.

Eine deutliche pharmakologische Wirkung von nicht derivatisiertem Prolin ist nicht bekannt und war aufgrund seines abundanten Vorkommens in der Natur auch nicht zu erwarten. Überraschenderweise wurde nun festgestellt, dass Prolin eine signifikante antiphlogistische, antirheumatische und analgetische Wirkung aufweist.

Die Erfindung betrifft daher die Verwendung von Prolin und seinen pharmazeutisch verträglichen Salzen zur Herstellung von Arzneimitteln, die Prolin als einzigen Wirkstoff enthalten, zur Behandlung von rheumatischen Erkrankungen und chronischen und traumatisch bedingten Schmerzzuständen. Die unter Verwendung von Prolin hergestellten Arzneimittel eignen sich insbesondere zur Anwendung als antiphlogistische, antirheumatische oder analgetische Mittel, sowie als Arzneimittel zur Behandlung von Entzündungs- oder Schmerzzuständen, insbesondere als Arzneimittel zur Behandlung von entzündlich bedingten, rheumatischen oder nicht-rheumatischen Schmerzzuständen oder von postoperativen oder posttraumatischen Schmerzen, wobei die Arzneimittel dadurch gekennzeichnet sind, dass sie eine therapeutisch wirksame Menge an Prolin oder einem seiner pharmazeutisch verträglichen Salze enthalten.

Im Rahmen der vorliegenden Erfindung bedeutet Prolin vorzugsweise das L-Prolin Erfindungsgemäss kommen beliebige Formen von entzündlich bedingten Schmerzzuständen, sowohl des rheumatischen als auch des nicht-rheumatischen Formenkreises (und damit assoziierte Bewegungseinschränkungen, wie z.B. eine Epicondylitis) sowie von postoperativen und posttraumatischen Schmerzen in Betracht. Obwohl der Wirkungsmechanismus noch nicht geklärt ist, zeigen sich die entzündungshemmenden und schmerzlindernden Eigenschaften klinisch bei rheumatischen Erkrankungen und Sporttraumen in einer deutlichen Besserung der Beschwerden, wie Ruheschmerz, Schmerz bei Bewegung, Belastungsschmerzen, Morgensteifheit, Schwellung der Gelenke, sowie in der Zunahme der Funktionsfähigkeit. Bei posttraumatischen und postoperativen Entzündungen und Schmerzen bewirken die erfindungsgemässen Substanzen eine rasche Abnahme von Spontan- und Bewegungsschmerzen und eine Verminderung der entzündlichen Schwellungen und der Ödembildung. Als ausgewählte Beispiele erfindungsgemässer Indikationen und Therapien können folgende Anwendungen erwähnt werden:
- entzündliche und degenerative Formen des Rheumatismus: chronische Polyarthritis, juvenile chronische Polyarthritis, Spondylitis ankylosans, Arthrosen einschliesslich Spondylarthrosen,
- schmerzhafte Wirbelsäulensyndrome,
- extraartikulärer Rheumatismus,
- schmerzhafte Entzündungs- und Schwellungszustände nach Traumen und operativen Eingriffen, z.B. in der Orthopädie,
- Iokalisierte Formen des Weichteilrheumatismus wie z.B. Tendovaginitis, Schulter-Hand-Syndrom, Bursitis,
- Iokalisierte rheumatische Erkrankungen wie z.B. Arthrosen peripherer Gelenke und der Wirbelsäule, Periarthritis,
- traumatisch und/oder belastungsbedingte Entzündungen der Sehnen, Bänder, Muskeln und Gelenke, wie z.B. Verstauchungen, Prellungen, Zerrungen.

Da L-Prolin eine natürliche Substan ist und in der Natur in grossen Mengen vorkommt, ist seine Verabreichung unproblematisch: toxische Effekte wie Nebenwirkungen und Kontraindikationen sind bisher nicht bekannt und auch nicht zu erwarten. Im Vergleich zu den klassisch-chemischen Analgetika und Antiphlogistika erlaubt Prolin eine rasche, hochwirksame und bessere - weil nebenwirkungsfreie - Therapie und somit auch eine Verbesserung der Patienten-Compliance.

Prolin kann gemäss vorliegender Erfindung als solches oder in Form seiner pharmazeutisch verträglichen Salze, beispielsweise in Form des Hydrochlorids oder des Natrium-, Calcium- oder Magnesiumsalzes, zur Herstellung von Arzneimitteln verwendet werden. Besonders wertvolle Salze sind solche, welche als saure Komponente Vitaminsäuren, wie beispielsweise Ascorbinsäure, Vitamin-A-säure usw., enthalten. Der Wirkstoffgehalt kann in einem breiten Bereich variieren und beispielsweise etwa 1 bis 99 Gew.-% des Arzneimittels betragen.

Prolin, und seine pharmazeutisch verträglichen Salze eignen sich zur Anwendung in allen für die genannten Indikationen oder Indikationsgruppen üblichen galenischen Formen. Solche pharmazeutischen Darreichungsformen sind beispielsweise im Magensaft zerfallende Tabletten, Dragées, Kapseln oder andere feste Zubereitungen, sogenannte osmotische Pumpsysteme, ein- oder mehrschichtige feste Zubereitungen, die eine verzögerte oder stufenweise Wirksstofffreisetzung gewährleisten, Pellets in Kapseln oder gepresst mit sofortiger oder verzögerter Wirksstofffreisetzung, magensaftresistente feste Zubereitungen, Lösungen der Wirksubstanzen in Weichgelatinekapseln oder nach speziellen Methoden versiegelten Hartgelatinekapseln oder anderen Umhüllungen, in Wasser oder anderen Getränken lösliche Formen, wie zum Beispiel Brausetabletten, Brausegranulate, Lösungstabletten und Lösungsgranulate, flüssige Zubereitungen wie Tropfen oder Sirupe zur Einnahme als Konzentrat oder verdünnt in Wasser oder anderen Getränken, transdermale Formen zur topischen Applikation wie Pflaster, Gele, Crèmes und Salben, flüssige Darreichungsformen zur Injektion und Infusion, Suppositorien oder andere Zubereitungen zur rektalen Applikation.

Bevorzugt sind feste oder halbfeste Verabreichungsformen, wie Tabletten, Dragées, Kapseln, Granulate, Suppositorien, Gele, Crèmes oder Salben, und flüssige Verabreichungsformen, wie Lösungen oder Suspensionen.

Bei der Herstellung der pharmazeutischen Präparate können konventionelle Misch-, Granulier-, Dragier-, Lösungs- oder Lyophilisierungsverfahren zur Anwendung kommen.

Pharmazeutische Präparate zur oralen Anwendung werden beispielsweise erhalten, indem man den Wirkstoff mit festen Trägerstoffen kombiniert, das erhaltene Gemisch gegebenfalls granuliert, und das Gemisch oder Granulat, falls gewünscht oder notwendig, nach Zugabe geeigneter Hilfsstoffe zu Tabletten oder Dragée-Kernen verarbeitet.

Geeignete Trägerstoffe sind insbesondere Füllstoffe, wie Zucker, zum Beispiel Lactose, Saccharose, Mannit oder Sorbit, Cellulosepräparate und/oder Calciumphosphate, zum Beispiel Tricalciumphosphat oder Calciumhydrogenphosphat, ferner Bindemittel, wie Stärkekleister unter Verwendung von zum Beispiel Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Als Hilfsmittel eignen sich in erster Linie Fliessregulier- und Schmiermittel, zum Beispiel Kieselsäure, Siliziumdioxid, Talk, Stearinsäure, oder Salze davon, wie Magnesium- oder Calciumstearat, und/oder Polyethylenglykol.

Weitere, oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, zum Beispiel im Gemisch mit Füllstoffen, wie Lactose, Bindemitteln, wie Stärken, und/oder Gleitmitteln, wie Talk oder Magnesiumstearat, und gegebenenfalls Stabilisatoren enthalten. In weichen Kapseln ist der Wirkstoff vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können.

Zur parenteralen Verabreichung eignen sich in erster Linie wässrige Lösungen eines Wirkstoffes in wasserlöslicher Form, ferner Suspensionen des Wirkstoffs, wie entsprechende ölige Injektionssuspensionen, wobei man geeignete lipophile Lösungsmittel oder Vehikel, wie fette Öle, zum Beispiel Sesamöl, oder Fettsäureester, zum Beispiel Ethyloleat oder Triglyceride, verwendet, oder wässrige Injektionssuspensionen, welche viskositätserhöhende Stoffe, zum Beispiel Natriumcarboxymethylcellulose, Sorbit und/oder Dextran und gegebenfalls auch Stabilisatoren enthalten.

Pharmazeutische Präparate zur topischen Anwendung sind Gele oder Hydrogele, Crèmes und Salben. Der Wirkstoff kann gelöst oder suspendiert in der Grundlage vorliegen. Im pharmazeutischen Sprachgebrauch ist es üblich die Bezeichnung "Salbe" für alle streichfähigen, halbfesten Zubereitungen zur Anwendung auf der Haut zu verwenden. In den folgenden Darlegungen wird der Begriff "Salbe" in diesem übergeordneten Sinne verwendet. Salben setzen sich aus der Salbengrundlage, die ein einfaches System (z.B. Vaselin) oder von komplexer Zusammensetzung (z.B. emulgatorhaltige Systeme) sein kann, und dem Wirkstoff zusammen.

Geeignete Salbengrundlagen enthalten beispielsweise Vaselin, Paraffin, Polyethylen, natürliche, hydrierte oder synthetische Fette (Triglyceride), Polyethylenglykole, Polyethylenoxide, Macrogole, Carbowachse, Wollwachs, Wollwachsalkohole, Cellulose, Cellulosederivate (z.B. Celluloseether), hochdisperses Siliziumdioxid, Bentonit, Stärken, Amylopectine, Amylopectinderivate, Alginate, Tragant, Polyacrylsäure, Polyvinylalkohol und/oder Polyvinylpyrrolidon. Geeignete Emulgatoren sind beispielsweise Cetylstearylalkohol, Cetylesteralkohol, Natriumlaurylsulfat, Natriumcetylsulfat, Natriumstearylsulfat, Natriumcetylsulfonat, Sorbitanester, Polysorbate und Polyoxyethylenfettalkoholether. Beispiele geeigneter Konservierungsstoffe sind Ethanol, Isopropanol, Sorbinsaure, Paraben (4-Hydroxybenzoesäure), Parabenester (4-Hydroxybenzoesäureester), Methylparaben, Propylparaben, Hexachlorophen, Benzalkoniumbromid Cetylpyridiniumchlorid und Ascorbinsäure. Geeignete Sorptionsvermittler (auch als Penetrationsvermittler, Enhancer oder Absorptionsbeschleuniger bezeichnet) sind beispielsweise Isopropylmyristat, Dimethylsulfoxid, 2-Pyrrolidon, 1-Dodecylazacycloheptan-2-on, *1,2-*Propylenglycol, Ölsäure, Natriumlaurylsulfat, Harnstoff, Salicylsäure, Hyaluronidase, Oleylalkohol und Ethylenglykol.

Die Dosierung des Wirkstoffes hängt von der zu behandelnden Krankheit, dem Körpergewicht, Alter und dem individuellen Zustand des Patienten sowie von der Applikationsart ab und wird am besten vom behandelnden Arzt eingestellt. Zur oralen und rektalen Applikation eignen sich vorzugsweise Zubereitungen mit 50 - 5000 mg Prolin bzw. eines seiner pharmazeutisch verträglichen Salze pro Einheit, die ein- bis viermal täglich eingenommen werden. Zur topischen Applikation eignen sich halbfeste oder flüssige Zubereitungen oder Patches mit einem Gehalt von 1 - 50 % Prolin bzw. eines seiner pharmazeutisch verträglichen Salze.

In zwei klinischen Voruntersuchungen wurden zwei Patientengruppen mit chronisch entzündlich oder traumatisch bedingten Schmerzzuständen während 5 Tagen mit einem oral bzw. topisch verabreichbaren Prolin-Präparat behandelt. Die erste Gruppe erhielt dreimal täglich eine Tablette mit 500 mg Prolin (hergestellt nach Beispiel 1). Die zweite Gruppe erhielt ein Gel enthaltend 5 Gewichtsprozent Prolin (hergestellt nach Beispiel 2), welches zwei- bis dreimal täglich auf die schmerzende Stelle aufgetragen wurde. Die Patienten wurden gebeten, täglich die Schmerzstärke auf einer Skala von 0 (kein Schmerz) bis 9 (sehr starker Schmerz> anzugeben. Die registrierten Absolutwerte und die normalisierten Werte (100 % entspricht dem Ausgangswert am Tag 0) sind in den Tabellen 1 - 4 zusammengestellt.

**Tabelle 1**

| Tablette mit 500 mg Prolin, dreimal täglich Registrierte Schmerzstärke auf einer Skala von 0 bis 9 | | | | | | |
|---|---|---|---|---|---|---|
| **Tag** | **0** | **1** | **2** | **3** | **4** | **5** |
| Patient 1 | | | | | | |
| Achillodynie | 7 | 7 | 7 | 6 | 4 | 3 |

| Patient 2 | | | | | | |
|---|---|---|---|---|---|---|
| Myogelose + Muskelschmerzen, Bein rechts | 7 | 7 | 8 | 7 | 5 | 5 |

| Patient 3 | | | | | | |
|---|---|---|---|---|---|---|
| Tarsaltunnelsyndrom | 9 | 9 | 9 | 5 | 4 | 5 |

| Patient 4 | | | | | | |
|---|---|---|---|---|---|---|
| Aduktorenansatz rechts gereizt | 3 | 3 | 2 | 1 | 1 | 1 |

| Patient 4 | | | | | | |
|---|---|---|---|---|---|---|
| Achillessehne/Soleus links gereizt | 2 | 2 | 1 | 0 | 0 | 0 |

| Patient 5 | | | | | | |
|---|---|---|---|---|---|---|
| Logendruck | 4 | 4 | 4 | 4 | 3 | 2 |
| **Mittelwert** | **5,3** | **5,3** | **5,2** | **3,8** | **2,8** | **2,7** |

**Tabelle 2**

| Tablette mit 500 mg Prolin, dreimal täglich Normalisierte Schmerzstärke in % | | | | | | |
|---|---|---|---|---|---|---|
| **Tag** | **0** | **1** | **2** | **3** | **4** | **5** |
| Patient 1 | | | | | | |
| Achillodynie | 100,0 | 100,0 | 100,0 | 85,7 | 57,1 | 42,9 |

| Patient 2 | | | | | | |
|---|---|---|---|---|---|---|
| Myogelose + Muskelschmerzen, Bein rechts | 100,0 | 100,0 | 114,3 | 100,0 | 71,4 | 71,4 |

| Patient 3 | | | | | | |
|---|---|---|---|---|---|---|
| Tarsaltunnelsyndrom | 100,0 | 100,0 | 100,0 | 55,6 | 44,4 | 55,6 |

| Patient 4 | | | | | | |
|---|---|---|---|---|---|---|
| Aduktorenansatz rechts gereizt | 100,0 | 100,0 | 66,7 | 33,3 | 33,3 | 33,3 |

| Patient 4 | | | | | | |
|---|---|---|---|---|---|---|
| Achillessehne/Soleus links gereizt | 100,0 | 100,0 | 50,0 | 0 | 0 | 0 |

| Patient 5 | | | | | | |
|---|---|---|---|---|---|---|
| Logendruck | 100,0 | 100,0 | 100,0 | 100,0 | 75,0 | 50,0 |
| **Mittelwert** | **100,0** | **100,0** | **88,5** | **62,4** | **46,9** | **42,2** |

**Tabelle 3**

| Gel mit 5 Gew.-% Prolin, 2-3 mal täglich Registrierte Schmerzstärke auf einer Skala von 0 bis 9 | | | | | | |
|---|---|---|---|---|---|---|
| **Tag** | **0** | **1** | **2** | **3** | **4** | **5** |
| Patient 1 | | | | | | |
| Knieschmerzen | 5 | 5 | 3 | 3 | 4 | 2 |

| Patient 2 | | | | | | |
|---|---|---|---|---|---|---|
| Achillodynie rechts | 5 | 5 | 4 | 4 | 3 | 2 |

| Patient 2 | | | | | | |
|---|---|---|---|---|---|---|
| Achillodynie links | 5 | 5 | 4 | 4 | 3 | 2 |

| Patient 3 | | | | | | |
|---|---|---|---|---|---|---|
| Achillodynie morgens | 7 | 7 | 6 | 3 | 1 | 3 |

| Patient 3 | | | | | | |
|---|---|---|---|---|---|---|
| Achillodynie abends | 5 | 5 | 3 | 1 | 2 | 2 |

| Patient 4 | | | | | | |
|---|---|---|---|---|---|---|
| Ellbogenluxation rechts | 4 | 4 | 3 | 2 | 1 | 2 |

| Patient 4 | | | | | | |
|---|---|---|---|---|---|---|
| Hals-Wirbelsäulenschmerzen nach Unfall | 6 | 4 | 3 | 2 | 1 | 1 |

| Patient 4 | | | | | | |
|---|---|---|---|---|---|---|
| Schulter links | 7 | 7 | 8 | 7 | 4 | 3 |

| Patient 5 | | | | | | |
|---|---|---|---|---|---|---|
| Bursitis subachillea rechts | 4 | 3 | 3 | 3 | 2 | 2 |

| Patient 6 | | | | | | |
|---|---|---|---|---|---|---|
| Ellbogenluxation links | 3 | 3 | 3 | 2 | 1 | 1 |

| Patient 7 | | | | | | |
|---|---|---|---|---|---|---|
| Achillodynie | 4 | 4 | 5 | 3 | 3 | 2 |
| **Mittelwert** | **5,0** | **4,7** | **4,1** | **3,1** | **2,3** | **2,0** |

**Tabelle 4**

| Gel mit 5 Gew.-% Prolin, 2-3 mal täglich Normalisierte Schmerzstärke in % | | | | | | |
|---|---|---|---|---|---|---|
| **Tag** | **0** | **1** | **2** | **3** | **4** | **5** |
| Patient 1 | | | | | | |
| Knieschmerzen | 100,0 | 100,0 | 60,0 | 60,0 | 80,0 | 40,0 |

| Patient 2 | | | | | | |
|---|---|---|---|---|---|---|
| Achillodynie rechts | 100,0 | 100,0 | 80,0 | 80,0 | 60,0 | 40,0 |

| Patient 2 | | | | | | |
|---|---|---|---|---|---|---|
| Achillodynie links | 100,0 | 100,0 | 80,0 | 80,0 | 60,0 | 40,0 |

| Patient 3 | | | | | | |
|---|---|---|---|---|---|---|
| Achillodynie morgens | 100,0 | 100,0 | 85,7 | 42,9 | 14,3 | 42,9 |

| Patient 3 | | | | | | |
|---|---|---|---|---|---|---|
| Achillodynie abends | 100,0 | 100,0 | 60,0 | 20,0 | 40,0 | 40,0 |

| Patient 4 | | | | | | |
|---|---|---|---|---|---|---|
| Ellbogenluxation rechts | 100,0 | 100,0 | 75,0 | 50,0 | 25,0 | 50,0 |

| Patient 4 | | | | | | |
|---|---|---|---|---|---|---|
| Hals-Wirbelsäulenschmerzen nach Unfall | 100,0 | 66,7 | 50,0 | 33,3 | 16,7 | 16,7 |

| Patient 4 | | | | | | |
|---|---|---|---|---|---|---|
| Schulter links | 100,0 | 100,0 | 114,3 | 100,0 | 57,1 | 42,9 |

| Patient 5 | | | | | | |
|---|---|---|---|---|---|---|
| Bursitis subachillea rechts | 100,0 | 75,0 | 75,0 | 75,0 | 50,0 | 50,0 |

| Patient 6 | | | | | | |
|---|---|---|---|---|---|---|
| Ellbogenluxation links | 100,0 | 100,0 | 100,0 | 66,7 | 33,3 | 33,3 |

| Patient 7 | | | | | | |
|---|---|---|---|---|---|---|
| Achillodynie | 100,0 | 100,0 | 125,0 | 75,0 | 75,0 | 50,0 |
| **Mittelwert** | **100,0** | **94,7** | **82,3** | **62,1** | **46,5** | **40,5** |

Wie die Ergebnisse der Untersuchungen zeigen, bewirken beide Präparate eine überraschend schnelle Verminderung von chronischen Schmerzzuständen.

Die vorliegende Erfindung wird durch die folgenden Beispiele weiter veranschaulicht. Avicel PH 102 (mikrokristalline Cellulose; FMC Corp.), Explotab (Natriumstärkeglykolat; Mendell, Patterson, New York), Syloid 244 (Silicalgel; W.R. Grace/Davison Chemical Div., Baltimore), Cutina HR (Wachsgemisch, ethoxylierte Ester; Henkel), Polysorbat 80 (Polyoxyethylensorbitanester; ICI), Carbopol 980 (Polyacrylsäure; BFGoodrich), PVP K30 (Polyvinylpyrrolidon bzw. Polyvidon; GAF, Grossbritannien), Eudragit (Acrylharz; Röhm, Deutschland) und Natrosol 250 HX (Hydroxyethylcellulose; Aqualon, Grossbritannien) sind bekannte Handelsprodukte. Temperaturen sind in Celsiusgraden angegeben.

### Beispiel 1: Tablette

Tabletten, enthaltend 500 mg Prolin, können wie folgt hergestellt werden:

| Zusammensetzung für 1 bzw. 1000 Tabletten: | | |
|---|---|---|
| | mg/Tablette | g/1000 Tabletten |
| Prolin | 500,0 | 500,0 |
| Avicel PH 102 | 86,0 | 86,0 |
| Explotab | 50,6 | 50,6 |
| Syloid 244 | 3,2 | 3,2 |
| Cutina HR | 5,2 | 5,2 |

Prolin, Avicel und Explotab werden innert 10 Minuten homogen vermischt. Anschliessend werden Syloid und Cutina innert 1 Minute daruntergemischt. Die fertige Mischung wird zu Tabletten verpresst (Gewicht: 645 mg).

### Beispiel 2: Gele

Gele, enthaltend 5% Prolin bzw. 4-Hydroxyprolin als Wirkstoff, können wie folgt hergestellt werden:

| Zusammensetzung für 1 kg Gel: | |
|---|---|
| | g/kg Gel |
| Wirkstoff | 50,0 |
| Isopropylmyristat | 20,0 |
| Polysorbat 80 | 60,0 |
| Carbopol 980 | 8,0 |
| Isopropanol | 380,0 |
| Wasser, ger. | 442,0 |
| NaOH 1N | 40,0 |

### Teilprodukt 1:

Isopropylmyristat und Polysorbat werden homogen vermischt. Dann wird Isopropanol dazugemischt. Wenn die Mischung homogen ist, wird unter Rühren Carbopol langsam dazugegeben und anschliessend 10 Minuten weitergerührt.

### Teilprodukt 2:

Der Wirkstoff wird in Wasser gegeben und so lange gerührt, bis sich der Wirkstoff vollständig aufgelöst hat.

### Fertigstellung des Gels:

Unter Rühren wird das Teilprodukt 2 zum Teilprodukt 1 gegeben und die Mischung 10 Minuten weitergerührt. Das Ansatzgefäss wird verschlossen und über Nacht stehen gelassen. Am nächsten Tag wird der Ansatz 5 Minuten gerührt, dann unter Rühren mit 1 N NaOH in dünnem Strahl versetzt (der pH des Gels soll zwischen 6 und 7 liegen) und 15 Minuten weitergerührt. Das Gel kann anschliessend in geeignete Behälter, z.B. Tuben aus Aluminium, abgefüllt werden.

### Beispiel 3: Retardtabletten

Retardtabletten, enthaltend 200 mg Prolin, können wie folgt hergestellt werden:

| Zusammensetzung für 1 bzw. 1000 Retardtabletten | | |
|---|---|---|
| | mg/Tablette | g/1000 Tabletten |
| Prolin | 200 | 200 |
| PVP K30 | 125 | 125 |
| CaHPO₄ | 25 | 25 |
| Eudragit RS | 131 | 131 |
| Natrosol 250 HX | 100 | 100 |

Zur Herstellung einer Granulierlösung für 1000 Retardtabletten werden 92 g Isopropanol und 62 g Aceton eingewogen und unter Rühren homogen vermischt. Anschliessend werden unter stetigem Rühren 6 g Eudragit RS eingestreut, und es wird weiter gerührt, bis alles gelöst ist.

Zur Herstellung eines Wirkstoffgranulates für 1000 Retardtabletten werden 200 g Prolin, 125 g PVP K30, 25 g Calciumhydrogenphosphat, 125 g Eudragit RS und 100 g Natrosol innert 5 Minuten homogen vermischt. Die Pulvermischung wird durch ein 0,7 mm-Sieb geschlagen und abermals während 5 Minuten im Mischer homogenisiert. Anschliessend wird die Pulvermischung mit der Granulierlösung angefeuchtet und homogen vermischt. Die feuchte Mischung wird durch ein 0,7 mm-Sieb geschlagen und bei 45°C getrocknet. Die trockene Massen wird nochmals durch 0,7 mm-Sieb geschlagen. Die fertige Mischung wird zu Tabletten verpresst (Gewicht: 581 mg).

## Patentansprüche

1. Verwendung von Prolin und seinen pharmazeutisch verträglichen Salzen zur Herstellung von Arzneimitteln, die Prolin als einzigen Wirkstoff enthalten, zur Behandlung von rheumatischen Erkrankungen und chronischen und traumatisch bedingten Schmerzzuständen.

2. Verwendung nach Anspruch 1 von L-Prolin und seinen pharmazeutisch verträglichen Salzen zur Herstellung von Arzneimitteln, die L-Prolin als einzigen Wirkstoff enthalten, zur Behandlung von rheumatischen Erkrankungen und chronischen und traumatisch bedingten Schmerzzuständen.

3. Verwendung von Prolin und seinen pharmazeutisch verträglichen Salzen nach einem der Ansprüche 1 oder 2 zur Herstellung von Arzneimitteln, die Prolin als einzigen Wirkstoff enthalten, zur Behandlung von rheumatischen Erkrankungen.

4. Verwendung von Prolin und seinen pharmazeutisch verträglichen Salzen nach einem der Ansprüche 1 oder 2 zur Herstellung von Arzneimitteln, die Prolin als einzigen Wirkstoff enthalten, zur Behandlung von chronischen und traumatisch bedingten Schmerzzuständen.

5. Verwendung von Prolin und seinen pharmazeutisch verträglichen Salzen nach einem der Ansprüche 1 bis 4 zur Herstellung von Arzneimitteln, die Prolin als einzigen Wirkstoff enthalten, in Form von in Form von Tabletten, Dragées, Kapseln, Granulaten, Suppositorien oder in Form eines Gels, einer Crème, einer Salbe oder einer Lösung oder Suspension.

## Claims

1. Use of proline and its pharmaceutically compatible salts for the manufacture of medicaments containing proline as sole active component for the treatment of rheumatic diseases and chronic and traumatic pain conditions.

2. Use according to claim 1 of L-proline and its pharmaceutically compatible salts for the manufacture of medicaments containing L-proline as sole active component for the treatment of rheumatic diseases and chronic and traumatic pain conditions.

3. Use of proline and its pharmaceutically compatible salts according to one of claims 1 or 2 for the manufacture of medicaments containing proline as sole active component for the treatment of rheumatic diseases.

4. Use of proline and its pharmaceutically compatible salts according to one of claims 1 or 2 for the manufacture of medicaments containing proline as sole active component for the treatment of chronic and traumatic pain conditions.

5. Use of proline and its pharmaceutically compatible salts according to one of claims 1 to 4 for the manufacture of medicaments containing proline as sole active component, in the form of tablets, dragées, capsules, granulates, suppositories or in the form of a gel, a cream, an ointment or a solution or suspension.

## Revendications

1. Utilisation de Prolin et de son sel pharmaceutiquement accepté pour la réalisation de médicaments qui contiennent le Prolin comme substance active unique, qui traite des maladies rhumatisantes, chroniques et traumatiques dûes à des douleurs persistantes.

2. Utilisation après la demande 1 du L-Prolin et de son sel pharmaceutiquement accepté pour la réalisation de médicaments qui contiennent le Prolin comme substance active unique qui traite des maladies rhumatisantes, chroniques et traumatiques dûes à des douleurs persistantes.

3. Utilisation de Prolin et de son sel pharmaceutiquement accepté après la demande 1 ou 2 pour la réalisation de médicaments qui contiennent le Prolin comme substance active unique qui traite des maladies rhumatisantes.

4. Utilisation de Prolin et de son sel pharmaceutiquement accepté après la demande 1 ou 2 pour la réalisation de médicaments qui contiennent le Prolin comme substance active unique qui traite des maladies rhumatisantes, chroniques et traumatiques dûes à des douleurs persistantes.

5. Utilisation de Prolin et de son sel pharmaceutiquement accepté après les demandes 1 à 4 pour la réalisation de médicaments qui contiennent le Prolin comme substance active unique que l'on peut trouver sous forme de tablettes, dragées, capsules, granulés, suppositoires, ou sous forme de gel, de crème, de pommade ou encore de solution ou suspension.
